# FASCICULE DE BREVET EUROPEEN

(11) **EP 0 721 510 B1**
(45) Date de publication et mention de la délivrance du brevet: **20.08.2003**
(21) Numéro de dépôt: 94928924.3
(22) Date de dépôt: 28.09.1994
(51) Int. Cl.: C12N 15/86, A61K 48/00, C12N 15/24, C12N 15/26, C07K 14/55, C07K 14/54

(54) **THERAPIE GENIQUE ANTICANCEREUSE PAR MODULATION DE LA REPONSE IMMUNITAIRE ET/OU INFLAMMATOIRE**
ANTITUMOR-GENTHERAPIE BEI IMMUNO-UND/ODER ENTZUENDUNGMODULATION
ANTI-CANCER GENE THERAPY BY MODULATION OF IMMUNE OR INFLAMMATORY RESPONSE

(30) Priorité: 29.09.1993 FR 9311601
(43) Date de publication de la demande: 17.07.1996
(60) Demande divisionnaire: 01110326.4
(73) Titulaire: TRANSGENE S.A., 67082 Strasbourg Cédex (FR)
(72) Inventeur: ACRES, Bruce, F-67000 Strasbourg (FR)
(74) Mandataire: Ahner, Francis
(86) Numéro de dépôt international: FR9401133
(87) Numéro de publication internationale: WO95009241

(56) Documents cités:
- WO-A-92/20356
- PROCEEDINGS OF THE NATIONAL ACADEMY OF SCIENCES OF USA., vol.90, no.10, 15 Mai 1993, WASHINGTON US pages 4645 - 4649 PLAUTZ, G. ET AL. 'Immunotherapy of malignancy by in vivo gene transfer into tumors' cité dans la demande

## Description

La présente invention concerne une méthode de traitement du cancer par thérapie génique. Elle a plus particulièrement pour objet l'usage d'un vecteur viral afin de délivrer au niveau des cellules tumorales, un gène codant pour un agent modulateur de la réponse immunitaire et/ou inflammatoire.

Il est généralement admis que le cancer est une maladie qui résulte d'une perte du contrôle de la multiplication cellulaire. Ses causes peuvent être multiples et dues notamment à un défaut de fonctionnement de gènes cellulaires (activation de gènes potentiellement oncogènes, par exemple par mutation(s) somatique(s) de gènes normaux ; dérégulation de l'expression de gènes cellulaires ; inhibition de l'expression de gènes suppresseurs de tumeurs) ou à l'expression indésirable de gènes viraux.

Au cours de ces 20 dernières années, il a été démontré que la plupart des cellules tumorales présentent à leur surface des antigènes spécifiques de tumeurs (antigènes du non-soi) qui ne trouvent pas leurs équivalents dans les cellules normales. Ces antigènes tumeur-spécifiques sont par exemple (i) des antigènes cellulaires dont l'expression survient pendant la période foeto-embryonnaire et régresse à la naissance jusqu'à disparaître, (ii) des antigènes qui s'expriment normalement à un très faible niveau et qui, exprimés à fort niveau, deviennent caractéristiques d'une tumeur ou (iii) des antigènes cellulaires dont la structure ou la conformation est modifiée.

En principe, l'expression aberrante de ces antigènes tumeur-spécifiques est susceptible de déclencher une réponse immunitaire du même type que celle induite par tout antigène du non-soi. Celle-ci met en oeuvre l'ensemble des cellules du système immunitaire dont les neutrophiles, lymphocytes, monocytes et macrophages.

D'une manière générale, il existe deux grands types de réponse immunitaire : la réponse de type humorale qui correspond à la production d'anticorps par les lymphocytes B et la réponse immunitaire à médiation cellulaire à effet cytotoxique, qui fait intervenir des cellules effectrices, essentiellement des lymphocytes T cytotoxiques (T_{c}), des cellules NK (pour Natural Killer en anglais) et des cellules phagocytaires. Des cellules régulatrices modulent les deux types de réponses immunitaires : essentiellement les lymphocytes T auxiliaires (T_{H} pour T helper en anglais) et les lymphocytes T suppresseurs (T_{S}).

Une réponse immunitaire est un phénomène extrêmement complexe qui requiert notamment la coopération de différents types cellulaires. Cette coopération s'effectue par l'intermédiaire des cytokines qui sont des molécules solubles qui interviennent en tant que médiateur de cellule à cellule.

En ce qui concerne l'immunité humorale, les lymphocytes B sont stimulés par des antigènes du non-soi clans leur conformation native. En réponse à cette stimulation, les lymphocytes B produisent des anticorps spécifiques dirigés contre ces antigènes étrangers.

Les lymphocytes T en revanche, ne peuvent être stimulés que par des peptides, produits de dégradation des antigènes du non-soi, présentés à la surface des cellules présentatrices d'antigènes (CPA) en association avec des antigènes du complexe majeur d'histocompatibilité (CMH).

L'activation des lymphocytes T a pour effet de déclencher leur amplification et la mise en oeuvre de leurs fonctions ; en particulier, la destruction des cellules infectées ou tumorales par les lymphocytes cytotoxiques.

Seule une classe d'antigènes désignée super-antigènes, n'est pas présentée de façon conventionnelle. En effet, les super-antigènes sont capables de se lier aux molécules du CMH sans être au préalable dégradés en peptides et peuvent activer simultanément une quantité de lymphocytes T supérieure à celle qui le serait par la voie des antigènes classiques. Ces super-antigènes sont donc susceptibles d'induire une forte réponse immunitaire.

L'inflammation est déclenchée par la réponse de l'organisme à l'égard d'une agression, par exemple une blessure ou une infection. La réponse inflammatoire comprend toute une série de réactions dont notamment la libération de molécules chimiotactiques ou chémoattractives (également désignées sous le terme chémokines) qui vont attirer les cellules du système immunitaire au site même de l'inflammation.

Les lymphocytes T activés produisent entre autres, des molécules inhibitrices des phénomènes de migration cellulaire, comme le MIF (pour Migration Inhibition Factor en anglais). Comme son nom l'indique, le MIF a pour rôle d'inhiber la migration des macrophages, et par conséquent, de favoriser leur concentration au niveau du site d'inflammation pour qu'ils exercent leur fonction de phagocytose dans des conditions optimales.

Dans le cas d'un cancer déclaré, la réponse immunitaire anti-tumorale est déficiente, soit parce que le système immunitaire lui-même est déficient, soit parce que les changements phénotypiques des cellules tumorales inhibent ou ne suffisent pas à déclencher la réponse immunitaire.

Afin de traiter les cancers, il a déjà été proposé de renforcer la réponse immunitaire anti-tumorale en administrant à des patients, des doses systémiques et répétées de cytokines telles que l'interleukine-2 (IL-2), l'interféron (IFNγ) ou le facteur nécrosant des tumeurs (TNF) de type α (Rosenberg, 1992, J. Clin. Oncology, *10*, 180-199). Malheureusement les effets secondaires sont non-négligeables, allant de la nausée voire même jusqu'à la mort. De plus, un tel traitement s'avère extrêmement coûteux.

Dans le même esprit, on a aussi proposé une méthode alternative basée sur le transfert *ex vivo* d'un gène codant pour une molécule immunostimulante, dans les cellules d'un patient ; ceci à des fins d'expression. Brièvement, (i) on prélève chez un patient, des cellules tumorales ou des lymphocytes infiltrant les tumeurs (TIL pour Tumor Infiltrating Lymphocyte en anglais) ; (ii) on les transfecte *ex vivo* par un vecteur portant un gène codant pour une molécule immunostimulante telle que l'IL-2, l'IL-4, l'IL-6 et le TNFα ; et (iii) on les réimplante chez le patient dont elles sont issues.

Comme précédemment, les essais cliniques n'ont jusqu'à présent donné que des résultats modestes, voire décevants. De nombreux expérimentateurs font état d'une faible expression (Anderson, 1993, Science, *259*, 1391-1392). De plus, un tel protocole est difficilement applicable à grande échelle. En effet, il nécessite la culture en masse de cellules pour chaque malade à traiter, avec les inconvénients que cela implique d'un point de vue coût, temps et risque. En outre, on ne peut garantir l'absence d'expression de nouveaux variants antigéniques tumoraux pendant la phase de culture *in vitro*.

Très récemment, Plautz et al (1993, Proc. Natl. Acad. Sci. USA, *90*, 4645-4649) ont reporté la transfection directe *in vivo* de cellules tumorales de souris par un rétrovirus recombinant. Ce dernier a été modifié afin de permettre l'expression d'un ADN complémentaire (ADNc) codant pour un antigène de surface murin du CMH. L'antigène retenu est allogénique, c'est-à-dire présentant une variation génétique par rapport à la souris hôte, dans le but de stimuler la réponse immunitaire à l'encontre des cellules tumorales exprimant cet antigène. Si cette méthode supprime le besoin d'établir une lignée cellulaire pour chaque malade, elle n'est cependant applicable qu'aux tumeurs accessibles par chirurgie.

On a maintenant trouvé qu'un virus de la vaccine administré à une souris développant une tumeur, infecte préférentiellement les tissus cancéreux. Lorsque le virus de la vaccine est porteur d'un gène codant pour une molécule immunostimulante, on observe une inhibition de la croissance des tumeurs et dans certains cas une régression complète.

WO92/20356 décrit l'utilisation de virus de la vaccine exprimant divers gènes codant pour des antigènes spécifiques de tumeur et des molécules agissant comme modulateur de la réponse immunitaire comme des cytokines ou des antigènes du complexe majeur d'histocompatibilité pour le traitement des cancers humains.

La présente invention a quant à elle pour objet l'usage d'un vecteur viral dérivé d'un virus de la vaccine dans le génome duquel est inséré un fragment d'ADN comportant un ou plusieurs gènes codant pour au moins un agent intervenant dans la destruction des cellules cancéreuses, e.g. un agent toxique pour les cellules cancéreuses ou un agent modulateur de la réponse immunitaire et/ou inflammatoire ; pour la préparation d'un médicament pour le traitement du cancer chez les mammifères, destiné à être administré par voie intraveineuse ou intramusculaire.

Par "vecteur viral", on entend un virus dont le génome a été modifié de manière à permettre le transfert et l'expression d'un gène d'intérêt dans une cellule eucaryote. Un vecteur viral pouvant être mis en oeuvre dans le cadre de la présente invention, peut être dérivé notamment d'un poxvirus, d'un virus de l'herpès, d'un rétrovirus ou d'un adénovirus. Avantageusement, il s'agira d'un vecteur non-intégratif, non-réplicatif et pour lequel la cellule-hôte d'origine est non-humaine, comme par exemple le poxvirus du canari. De tels vecteurs ainsi que leurs techniques de préparation sont connus de l'homme de l'art.

S'agissant d'un vecteur viral dérivé d'un adénovirus, celui-ci sera de préférence constitué par le génome complet de l'adénovirus au moins dépourvu du gène E1A situé à l'extrémité 5' et codant pour une protéine trans-activatrice essentielle à la réplication de l'adénovirus. Il sera donc propagé dans une lignée cellulaire de complémentation fournissant en *trans* le produit d'expression du gène E1A. Il va de soi que l'on peut modifier ou déléter d'autres régions du génome adénoviral, en particulier la région E3 non-essentielle et, de manière alternative, les autres régions essentielles à la réplication virale dans la mesure où les fonctions manquantes sont complémentées en *trans*. Un tel vecteur comprendra néanmoins les séquences essentielles à l'encapsidation, à savoir les ITRs (Inverted Terminal Repeat) 5' et 3' et la région d'encapsidation. Les différents vecteurs adénoviraux ainsi que leurs techniques de préparation sont conventionnels et sont présentés dans Graham et Prevect (Methods in Molecular Biology, vol *7*, p 109-128 ; Ed : E.J. Murey, The human Press Inc.).

Selon un mode particulièrement préféré, un vecteur viral utile aux fins de la présente invention est dérivé d'un poxvirus, notamment d'un virus de la vaccine ou d'un poxvirus aviaire, tel que le poxvirus du canari ; ce dernier étant préféré.

Les conditions générales d'obtention d'un virus de la vaccine capable d'exprimer un gène hétérologue sont décrites dans le brevet européen EP 83 286 et la demande EP 206 920. Selon un mode avantageux, ledit fragment d'ADN sera inséré dans le gène TK du virus de la vaccine, de façon à inactiver le gène viral et faciliter la sélection des virus de la vaccine recombinants.

Conformément aux buts poursuivis par la présente invention, un vecteur viral peut en outre comprendre un bloc d'expression d'un gène marqueur de sélection afin de faciliter les étapes d'isolement et de purification du virus recombinant. On peut citer notamment le gène *Neo* conférant la résistance à l'antibiotique G418 ou le gène TK du virus de l'herpès simplex de type 1 (HSV-1) qui confère la sensibilité à certains analogues de nucléosides tels que le ganciclovir ou l'acyclovir.

Un tel vecteur viral peut également inclure un gène autre que ceux définis ci-dessus qui peut agir de façon coopérative à l'effet modulateur de la réaction immunitaire et/ou inflammatoire recherché. Il s'agira avantageusement d'un gène codant pour tout ou partie d'un antigène spécifique de tumeurs. On peut citer plus particulièrement les protéines E6, E7 du virus HPV notamment de type 16 ou 18 impliquées dans les cancers de l'utérus, la protéine MUC1 et, plus particulièrement la région répetée de celle-ci, impliquée dans les cancers du sein et enfin l'antigène GA. 733.2 impliquée dans les cancers colorectaux. Les séquences codant pour ces antigènes sont décrites dans l'art antérieur. Il est à la portée de l'homme du métier de les isoler, par exemple par la technique du PCR (Polymérase Chain Reaction) en mettant en oeuvre des amorces complémentaires, de les insérer dans le vecteur viral retenu en amont ou en aval du gène modulateur et de les placer sous le contrôle des éléments nécessaires à leur expression.

Aux fins de la présente invention, un fragment d'ADN comportant un ou plusieurs gènes codant pour tout ou partie d'un agent modulateur de la réponse immunitaire et/ou inflammatoire, ci-après désigné agent modulateur, est introduit dans un vecteur viral, véhicule de transfert et d'expression du(des) dit(s) gène(s). Les méthodes pour insérer un fragment d'ADN dans un vecteur viral sont connues de l'homme du métier.

Par ailleurs, le gène codant pour un agent modulateur peut être de type génomique (comportant tout ou partie de l'ensemble des introns du gène naturel), de type ADN complémentaire (ADNc) dépourvu d'intron ou de type minigène c'est à dire mixte comportant au moins un intron. Il peut coder pour un agent modulateur natif tel que trouvé dans un mammifère, pour une partie de celui-ci, pour une molécule chimérique provenant de la fusion de séquences d'origine diverse ou un mutant présentant des propriétés biologiques améliorées ou modifiées, à la condition toutefois que ces molécules exercent une fonction immunomodulatrice ou modulatrice de la réponse inflammatoire. Un tel mutant peut être obtenu par mutation, délétion, substitution et/ou addition d'un ou plusieurs nucléotide(s) du gène codant pour ledit agent modulateur. Le gène en question peut coder pour (i) une molécule soluble, soit intracellulaire soit secrétée dans le milieu extérieur ou (ii) une molécule ancrée dans la membrane et donc présente à la surface des cellules qui l'expriment.

Les gènes codant pour un agent modulateur peuvent être obtenus par clonage, par PCR ou par synthèse chimique selon les techniques conventionnelles communément en usage.

Bien entendu, le fragment d'ADN peut comprendre les éléments appropriés de régulation de la transcription ainsi que des signaux d'initiation et de terminaison de la traduction permettant l'expression du ou des gène(s) codant pour un agent modulateur. Parmi ces éléments, la région promotrice revêt une importance particulière.

D'une façon générale, on aura recours à une région promotrice fonctionnelle dans les cellules du mammifère que l'on veut traiter, de préférence dans les cellules humaines. Il peut s'agir de la région promotrice gouvernant naturellement l'expression dudit gène ou d'une région promotrice d'origine différente, par exemple issue de gènes eucaryotes ou viraux. D'autre part, la région promotrice peut être modifiée de manière à contenir des séquences régulatrices, par exemple un élément activateur de la transcription (enhancer) ou des séquences répondant à certains signaux cellulaires.

La région promotrice retenue pourra être constitutive ou régulable, et dans ce dernier cas, régulable en réponse à certains signaux cellulaires, tissu-spécifiques ou événement-spécifiques. Il sera avantageux d'utiliser une région promotrice tissu-spécifique, lorsque la tumeur à traiter est issue d'un type cellulaire particulier. D'une manière alternative, l'utilisation d'un promoteur répondant à des signaux spécifiquement tumoraux (par exemple régulable par la présence de facteurs de croissance généralement libérés par les cellules tumorales) peut s'avérer avantageux puisque l'expression sera limitée aux cellules tumorales.

De tels promoteurs sont généralement connus de l'homme de l'art. On peut citer notamment les promoteurs SV40 (Virus Simian 40), HMG (Hydroxy-Methyl-Glutaryl-coenzyme A), TK (Thymidine Kinase), les LTRs (Long Terminal Repeat) du RSV (Rous Sarcoma Virus), du Mo-MLV (Moloney Murine Leukemia Virus), le MLP (Major Late Promoter) de l'adénovirus, les promoteurs 7,5K et H5R du virus de la vaccine, les promoteurs foie-spécifiques des gènes de l'αl- antitrypsine, de l'albumine, du facteur IX de la coagulation et de la transferrine et les promoteurs des gènes d'immunoglobulines qui permettent l'expression des gènes qu'ils gouvernent dans les lymphocytes. Ces exemples ne sont pas limitatifs.

Dans le cadre de la présente invention, un fragment d'ADN peut comporter un ou plusieurs gène(s) codant pour un agent modulateur qui peuvent être placés sous le contrôle des éléments permettant leur expression, de façon indépendante ou commune. En d'autres termes, le fragment d'ADN pourra contenir une ou plusieurs cassettes d'expression d'un ou plusieurs gènes codant pour un agent modulateur.

Par ailleurs, le fragment d'ADN peut également inclure une séquence signal permettant la sécrétion de l'agent modulateur en question en dehors de la cellule. Il peut s'agir de la séquence signal naturelle du gène codant pour ledit agent modulateur ou bien d'une séquence signal hétérologue fonctionnelle dans les cellules eucaryotes, par exemple la séquence signal du gène codant pour la transferrine ou l' α1-antitrypsine.

Par agent modulateur de la réponse immunitaire et/ou inflammatoire, on entend toute molécule notamment capable :
- de stimuler une réponse immunitaire humorale, en activant les lymphocytes B, afin d'amplifier la production d'anticorps dirigés contre les antigènes spécifiques de tumeurs ;
- de stimuler une réponse immunitaire à médiation cellulaire, en activant les lymphocytes T, afin de déclencher une réponse cytotoxique ou de type hypersensitivité retardée (DTH) significative à l'encontre des cellules tumorales ;
- d'induire ou de stimuler une réponse inflammatoire, afin de drainer les cellules du système immunitaire au niveau du site tumoral ; ou
- d'inhiber les phénomènes de migration cellulaire, afin de conserver les cellules du système immunitaire au site même ou à proximité d'un site tumoral.

Une molécule présentant une ou plusieurs des fonctions ci-dessus définies, peut être sélectionnée notamment parmi (I) les cytokines, (II) les molécules co-stimulantes de surface cellulaire, (III) les chémokines, (IV) les anticorps monoclonaux dirigés contre des marqueurs de surface lymphocytaire, (V) les super-antigènes caractéristiques d'un organisme infectieux (bactérie, virus ou parasite) et (VI) les polypeptides à fonction d'adjuvant.

### (I)

Des cytokines avantageuses aux fins de la présente invention, sont celles produites par les cellules du système immunitaire, notamment les lymphocytes et les macrophages ou bien leurs cellules souches progénitrices et qui interviennent dans l'activation des cellules du système immunitaire, le transport de signaux entre les cellules du système immunitaire et la différentiation cellulaire des cellules souches en cellules matures de la circulation sanguine.

A titre de cytokines utiles aux fins de présente invention, on cite notamment :
(1) Les interleukines (IL). A l'heure actuelle, 16 interleukines ont été identifiées. Il est particulièrement difficile de leur attribuer une fonction spécifique car elles exercent des effets pléïotropes. Dans le cadre de la présente invention, toutes les interleukines présentent un intérêt, mais on cite plus particulièrement :
   - L'IL-1, qui est produite par les macrophages à la suite d'une stimulation par des composants bactériens. Son rôle s'exerce au niveau de l'activation lymphocytaire. L'IL-1 est aussi une molécule pro-inflammatoire et à ce titre induit la production de chémokines.
   - L'IL-2, qui est responsable de la prolifération des lymphocytes T activés, et qui, en association avec l'IFNγ, stimule la production d'anticorps par les lymphocytes B. De plus, certaines études tendent à montrer qu'elle a un rôle chimiotactique pour les lymphocytes lorsqu'elle est produite au niveau d'une tumeur.
   - L'IL-4, qui est produite par les lymphocytes T activés et stimule la croissance des lymphocytes B et, dans certains cas, des lymphocytes T.
   - L'IL-5, qui favorise la multiplication et la différentiation des leucocytes éosinophiles ainsi que, dans une moindre mesure, la production des anticorps.
   - L'IL-6, qui est produite par les macrophages et les lymphocytes T. Elle a un effet pléïotropique et intervient entre autre en tant que stimulant de l'activité cytotoxique des lymphocytes T et de la production d'anticorps. Il s'agit également d'une molécule pro-inflammatoire.
   - L'IL-7, qui est produite par les cellules du stroma de la moelle osseuse et intervient dans la prolifération des lymphocytes pré-B et -T.
   - L'IL-12, qui est produite par les macrophages activés et induit la production d'IFNγ.
(2) Les interférons (IFN), qui possèdent des propriétés antivirales et immunomodulatrices. Ils peuvent activer les cellules phagocytaires et accroître l'expression des antigènes de surface de classe I et II du CMH et stimuler également la cytotoxicité des cellules NK à l'encontre des cellules tumorales. Il existe trois classes principales d'IFNs, chacune présentant un intérêt clans le cadre de la présente invention. Ces différentes classes, respectivement α, β et γ comprennent chacune de nombreux sous-types.
(3) Les facteurs stimulant les colonies CSF (pour Colony Stimulating Factor en anglais), qui interviennent au niveau de la maturation des cellules souches hématopoïétiques et leur différentiation en cellules matures de la circulation sanguine. On distingue les GM-CSF, G-CSF et M-CSF (pour Granulocyte-Macrophage, Granulocyte et Macrophage respectivement) selon le stade de maturation et le type cellulaire sur lequel ces facteurs exercent leur influence.
(4) Les facteurs nécrosant des tumeurs (TNF). On distingue le TNFα produit par les macrophages et le TNFβ produit par les lymphocytes T. Tous deux sont responsables de l'activité cytotoxique anti-tumorale des macrophages et lymphocytes et de l'altération tissulaire locale observée clans la réaction inflammatoire.
(5) Les facteurs MIF, qui inhibent la migration des macrophages.
(6) Le fragment C5a du complément, qui est un facteur chimiotactique des macrophages.

Aux fins de la présente invention, sont tout particulièrement préférés, l'IL-2, l'IL-4, l'IL-5, l'IL-6, l'IL-7, l'IFNγ, le GM-CSF et le TNFβ.

### (II)

Les molécules co-stimulantes sont des molécules présentes à la surface cellulaire qui participent d'une façon indirecte à la réaction immunitaire et/ou inflammatoire ; par exemple, des molécules intervenant dans le processus de présentation de l'antigène sous une forme reconnaissable par les cellules du système immunitaire.

A titre de molécules co-stimulantes utiles aux fins de la présente invention, on cite notamment :
(1) Les antigènes du complexe majeur d'histocompatibilité (CMH). Chez l'homme, la plupart des cellules nucléées expriment à leur surface des quantités variables d'antigènes de classe I, alors que les antigènes de classe II présentent une distribution restreinte aux lymphocytes B, cellules phagocytaires et lymphocytes T activés. Ces marqueurs de surface cellulaire sont impliqués dans les phénomènes de reconnaissance immune, notamment comme cela a été décrit précédemment, dans l'interaction entre lymphocytes T et cellules CPA et entre lymphocytes T_{c} et cellules cibles présentant à leur surface des antigènes du non-soi. On peut donc penser que l'intensité de la réponse immunitaire peut être améliorée en augmentant l'expression des antigènes du CMH au site tumoral.
(2) Les ligands des marqueurs humains CD27, CD28, CD30 et CD40. En particulier, le ligand du marqueur CD40 se situe à la surface des lymphocytes T activés et est impliqué dans la stimulation et la prolifération des lymphocytes B. D'autre part, des études *in vitro* ont montré que les ligands des marqueurs CD27 et CD30 induisent la prolifération des lymphocytes T. Quant au ligand du marqueur CD28, encore désigné par certains auteurs protéine B7, il est synthétisé par les CPA et intervient dans la reconnaissance des cellules cibles présentant des antigènes du non-soi à leur surface par les cellules NK permettant ainsi leur destruction.
(3) L'antigène de fonction lymphocytaire de type 1 LFA-1 (pour Leukocyte Function Antigen en anglais). Il s'agit d'un récepteur de surface commun à tous les leucocytes et également présent sur les macrophages, qui joue un rôle important dans les phénomènes d'adhésion cellulaire non-spécifique. Ceux-ci interviennent dans le processus de chimiotactisme en participant à la migration des cellules du système immunitaire vers un site inflammatoire.
(4) La molécule d'adhésion intercellulaire de type 1 (ICAM-1) présente à la surface de nombreux types cellulaires. ICAM-1 est un des ligands de la molécule LFA-1.

### (III)

Tel que précédemment énoncé, les chémokines sont des molécules synthétisées à proximité ou au niveau d'un site inflammatoire et qui drainent les cellules du système immunitaire vers ce site. D'une façon générale, les chémokines agissent par l'intermédiaire d'interactions entre d'une part, des molécules d'adhésion présentes à la surface des leucocytes et d'autre part des molécules d'adhésion présentes à la surface des cellules endothéliales (par exemple LFA-1 et ICAM-1). L'attachement des leucocytes aux cellules endothéliales de la paroi vasculaire est suivi de leur migration vers le site inflammatoire.

A titre de chémokines utiles aux fins de la présente invention, on cite notamment :
(1) Le PF-4 (pour Platelet Factor-4 en anglais ; Denel et al., 1977, Proc. Natl. Acad. Sci. USA, *74*, 2256) ;
(2) Le RANTES, facteur chimiotactique des différents types de leucocytes (Schall et al., 1988, J. Immunol., *141*, 1018) ; et
(3) L'ACT-2 (Ziptel et al., 1989, J. Immunol., *142*, 1587). Il s'agit d'une protéine qui favorise l'adhésion des lymphocytes T intervenant dans la reconnaissance de l'antigène associé aux molécules de classe I du CMH.

### (IV)

Un anticorps monoclonal utile aux fins de présente invention est par exemple, dirigé contre le marqueur de surface cellulaire CD2, CD3, CD28 ou CD40. En effet, certaines études ont montré le rôle d'un tel anticorps dans la stimulation de la prolifération des lymphocytes T.

### (V)

Un super-antigène utile aux fins de la présente invention est par exemple, la nucléoprotéine du virus de la rage ou une entérotoxine bactérienne, notamment produite par une bactérie du genre *Staphylococcus*.

### (VI)

Un adjuvant de nature polypeptidique, utile aux fins de la présente invention est par exemple, un produit d'expression d'un gène bactérien "layer" 20S.

Le médicament issu de la présente invention est destiné au traitement d'un cancer chez les mammifères, tout particulièrement chez les humains. Les cancers qui pourraient être ainsi traités sont, avantageusement, des tumeurs solides telles que les cancers du sein, du poumon et du colon. On indique qu'un tel médicament devrait plus particulièrement permettre de traiter les tumeurs secondaires qui sont des complications fréquentes rencontrées dans de nombreux types de cancers et pour lesquelles actuellement il n'existe pas de thérapie satisfaisante.

Le médicament issu de la présente invention peut être administré selon n'importe quelle voie générale communément en usage, notamment par voie parentérale telle que la voie systémique, intramusculaire, sous-cutanée ou intraperitonéale. D'une manière générale, la voie intraveineuse est indiquée comme étant particulièrement avantageuse. Alternativement, dans le cas d'une tumeur accessible, le médicament peut être également administré par injection directe dans le site de la tumeur ou par application topicale. D'une manière générale, l'administration peut avoir lieu en dose unique ou répétée, une ou plusieurs fois après un certain délai d'intervalle.

Selon un mode de mise en oeuvre préféré de l'invention, le médicament comprendra, outre une quantité thérapeutiquement efficace dudit vecteur viral, un support acceptable d'un point de vue pharmaceutique. Il peut également comprendre un véhicule, un diluant ou un adjuvant acceptable d'un point de vue pharmaceutique et se présenter sous forme liquide ou lyophilisée.

Le dosage approprié varie en fonction de différents paramètres, par exemple de la voie d'administration, de l'individu à traiter, de la nature et de la sévérité de l'état tumoral, du type de vecteur viral mis en oeuvre ou encore du ou des gène(s) codant pour l'agent modulateur. Un des critères qui permet d'évaluer le dosage approprié est la mesure de l'activité sérique de l'agent modulateur. Ces tests d'activité sont des tests standards. En particulier, on peut citer le test de bioactivité en IL-2 (Gillis et al, 1978, J. Immunol., *120*, 2027-2032). Cependant en général la dose de vecteur viral / kilo sera de 10⁴ à 10¹¹, avantageusement de 10⁷ à 10¹⁰ et de préférence de 10⁷ à 10⁹ unités formant des plages (ufp) / kilo.

Enfin, la présente invention a également pour objet :
(i) L'usage d'un vecteur viral présentant un tropisme positif pour les cellules cancéreuses, dans le génome duquel est inséré un fragment d'ADN comportant un ou plusieurs gènes codant pour au moins un agent intervenant dans la destruction des cellules cancéreuses, e.g. un agent modulateur de la réponse immunitaire et/ou inflammatoire, placé sous le contrôle des éléments nécessaires à l'expression, notamment pour délivrer de manière spécifique ledit agent au niveau d'une tumeur cancéreuse ;
(ii) Une méthode de traitement du cancer chez les mammifères, selon laquelle on injecte à un individu avant besoin d'un tel traitement, une quantité efficace d'un point de vue pharmaceutique d'un vecteur viral dans le génome duquel est inséré un fragment d'ADN comportant un ou plusieurs gènes codant pour au moins un agent intervenant dans la destruction des cellules cancéreuses, e.g. un agent modulateur de la réponse immunitaire et/ou inflammatoire ; et
(iii) Une méthode pour délivrer de manière spécifique un agent intervenant dans la destruction des cellules cancéreuses, e.g. un agent modulateur de la réponse immunitaire et/ou inflammatoire, selon laquelle on administre par voie générale un vecteur viral dans le génome duquel est inséré un fragment d'ADN comportant un ou plusieurs gènes codant pour tout ou partie dudit agent.

La présente invention est illustrée, sans pour autant être limitée, par les exemples suivants.

### EXEMPLES

### EXEMPLE 1 : Construction des virus de la vaccine recombinants porteurs d'un gène codant pour une cytokine.

Les constructions décrites ci-après sont réalisées selon les techniques générales de génie génétique et de clonage moléculaire détaillées dans Maniatis et al. (1989, Laboratory Manual, Cold Spring Harbor Laboratory Press, Cold Spring Harbor, NY). L'ensemble des étapes de clonage mettant en oeuvre des plasmides bactériens est effectué par passage dans la souche *Escherichia coli* (*E. coli*) 5K ou XL1-Blue (Stratagène), alors que celles mettant en oeuvre des vecteurs dérivés du phage M13 sont réalisées dans *E. coli* NM522.

En ce qui concerne les mutagénèses dirigées par oligodésoxynucléotides synthétiques, on applique le protocole décrit par Zoller et Smith (1982, Nucleic Acids Res., 10, 6487) ou on met en oeuvre un kit d'origine commerciale selon les recommandations du fabricant.

Les fragments d'ADN comportant les gènes codant pour le GM-CSF, IL-4, IL-5, IL-6 et IL-7, sont soumis à une étape de mutagénèse dirigée afin d'introduire les sites de restriction adéquats en vue de leur insertion dans un vecteur de transfert, en aval du promoteur 7,5K du virus de la vaccine. Deux vecteurs de transfert sont utilisés : pTG186-poly (décrit dans la demande de brevet EP 206 920) et pTG194. Ce dernier diffère de pTG186-poly par l'orientation du polylinker.

Plus précisément :

Un fragment portant l'ADNc codant pour le GM-CSF murin tel que décrit dans Gough et al. (1984, Nature, *309*, 763) est soumis à une mutagénèse dirigée après clonage dans M13TG130 (Kieny et al., 1983, Gene, *26*, 91), afin de créer un site *Bam*HI en position -17 par rapport à l'ATG initiateur de la traduction. Le fragment *Bam*HI-*Sal*I isolé du vecteur en résultant est inséré entre les mêmes sites de pTG194.

Un fragment *Eco*RI-*Bam*HI portant l'ADNc codant pour l'IL-4 murin tel que décrit dans Lee et al. (1986, Proc. Natl. Acad. Sci. USA, *83*, 2061) est introduit dans le vecteur M13TG130 et soumis à une mutagénèse dirigée, afin de créer un site *Bgl*II immédiatement en amont de l'ATG initiateur de la traduction. Le vecteur ainsi traité est digéré par *Eco*RI et *Bgl*II et le fragment correspondant introduit entre les mêmes sites de pTG194.

Un fragment *Eco*RI-*Bam*HI portant l'ADNc codant pour l'IL-5 murin tel que décrit dans Kinashi et al. (1986, Nature, *324*, 70) est tout d'abord sous cloné dans le vecteur M13TG130 puis soumis à une mutagénèse dirigée, afin d'introduire un site *Pst*I en position -10 et un A en position -3 par rapport à l'ATG initiateur de la traduction. Le vecteur ainsi traité est digéré par *Eco*RI et *Pst*I et le fragment correspondant inséré entre les mêmes sites de pTG186-poly.

Un fragment *Eco*RI portant l'ADNc codant pour l'IL-6 murin tel que décrit dans Van Snick et al. (1988, Eur. J. Immunol., *18*, 193) est sous cloné dans le vecteur M13TG131 (Kieny et al., *supra*) et soumis à une mutagénèse dirigée, afin d'introduire un site *Bam*HI en position -9 et un A en position -3 par rapport à l'ATG initiateur de la traduction. Le vecteur ainsi traité est digéré par *Eco*RI et *Bam*HI et le fragment correspondant inséré entre les sites *Bam*HI et *Eco*RI de pTG194.

Un fragment *Sat*I-*Hind*III portant l'ADNc codant pour l'IL-7 murin tel que décrit dans Naman et al. (1988, Nature, *333*, 571) est sous cloné dans le vecteur M13TG130 et soumis à une mutagénèse dirigée, afin de créer un site *Eco*RV en position -11 et un A en position -3 par rapport à l'ATG initiateur de la traduction. On isole un fragment *Eco*RV-*Pst*I du vecteur ainsi obtenu que l'on clone entre les sites *Sma*I et *Pst*I de pTG194.

A l'aide des vecteurs de transfert obtenus ci-dessus, on produit les virus de la vaccine correspondants selon la méthode de recombinaison homologue décrite par Kieny et al. (1984, Nature, *312*, 163). Les virus recombinants ainsi obtenus sont désignés VV-GM-CSF, VV-IL-4, etc.

D'autres virus de la vaccine recombinants ont été précédemment décrits ; en particulier ceux comportant l'ADNc codant (i) pour l'IL-2 humaine (demande de brevet EP 206 939), (ii) pour l'IL-6 humaine (Nakagawa et al., 1991, Eur. Cytokine Net., 2, 11-16) et (iii) pour l'IFNγ humain (demande de brevet EP 206 920).

### EXEMPLE 2 : Traitement de souris Swiss nude porteuses de tumeurs par un virus de la vaccine comportant le gène codant pour l'IL-6 (VV-IL-6)

### 1. Préparation des souris Swiss nude porteuses de tumeurs

La lignée cellulaire SW948 (ATCC CCL 237) générée à partir d'une tumeur humaine colorectale est cultivée en continu selon les recommandations du fournisseur. Les cellules que l'on récolte sont traitées par la trypsine puis par la désoxyribonucléase (10 µg/ml) pendant 5 min. Elles sont ensuite lavées clans du PBS (tampon phosphate salin Dulbecco ; Sigma, D5652) et resuspendues dans ce même tampon à la concentration de 10⁷ cellules / 100 µl.

Des souris femelles Swiss nude (Iffa Credo, France) âgées de 6 à 8 semaines reçoivent chacune 100 µl de la suspension cellulaire ainsi obtenue, par voie sous-cutanée. Sept jours plus tard, on sélectionne les souris porteuses de tumeurs palpables.

### 2. Test

Ces souris sont réparties en lots de huit environ. Deux lots sont destinés à servir de lots contrôle : les souris reçoivent soit 10⁷, soit 10⁸ ufp d'un virus de la vaccine non recombinant, TK-, généré à partir du vecteur de transfert pTG186-poly (VV-186). Les souris d'un troisième lot (lot-témoin négatif) reçoivent uniquement 100 µl de PBS. Enfin, les souris de deux autres lots reçoivent soit 10⁷, soit 10⁸ ufp du VV-IL-6 murin obtenu à l'Exemple 1.

Les virus sont administrés en solution de PBS, par voie intraveineuse au niveau de la queue.

Sept jours après les injections, on prélève 3 souris clans chacun des lots, et on les sacrifie afin d'analyser le contenu viral de leurs sang, organes et tumeurs, comme suit.

Les tumeurs et différents organes (foie, rate, cerveau, etc) une fois prélevés, sont traités à la trypsine et disloqués mécaniquement jusqu'à l'obtention d'une suspension. Le sang prélevé est dilué volume à volume dans du tampon PBS comprenant 20 mM d'EDTA.

Les cellules ainsi obtenues sont lavées deux fois dans du PBS, resuspendues dans du milieu de culture MEM Dubelcco (Modified Eagles Medium ; Gibco BRL) comprenant 10% de sérum foetal de veau. Le nombre des cellules (viables et non-viables) est estimé par comptage selon les méthodes classiques. Puis on effectue des dilutions en série de 10 en 10 dans du tampon PBS. On ajoute 1µl, 10 µl ou 100 µl de chacune des dilutions dans des cultures de cellules BHK établies en boîte de pétri de 3cm de diamètre (Falcon 3001). Les boîtes sont incubées toute la nuit à 37°C dans 5% de CO₂ et les plages de lyse dénombrées le jour suivant.

En ce qui concerne les souris non-sacrifiées, on enregistre l'évolution de la croissance des tumeurs au cours du temps, en mesurant la longueur, la largeur et la profondeur des tumeurs à l'aide d'un pied à coulisse. Le volume de chaque tumeur est calculé en appliquant la formule des ellipsoïdes 4/3πr₁r₂r₃, dans laquelle r₁, r₂ et r₃ représentent respectivement la longueur, largeur et profondeur réduites de moitié.

### 3. Résultats

Quelque soit le type de virus injecté, on constate que le taux d'infection des cellules tumorales est très supérieur à celui des tissus sains.

La croissance des tumeurs des souris ayant reçues le VV-IL-6, quelque soit la dose, est moindre par rapport à celles des lots témoins et du contrôle négatif. Des souris présentent une régression totale des tumeurs environ 15 jours après l'administration de VV-IL-6.

### EXEMPLE 3 : Traitement de souris DBA/2 porteuses de tumeurs par un virus de la vaccine comportant le gène codant pour IL-2 (VV-IL-2)

L'Exemple 2 est répété en utilisant :
(i) le virus de la vaccine comportant le gène codant pour l'IL-2 humaine, tel que décrit dans la demande de brevet EP 206 939 ;
(ii) la lignée cellulaire murine P815 (ATCC TIB 64) issue d'un mastocytome de souris DBA/2 ; et
(iii) des souris femelles DBA/2 (Iffa Credo, France) âgées de 6 à 8 semaines.

Les variantes sont comme suit : 10⁵ cellules P815 sous un volume de 100 µl sont injectées aux souris DBA/2. 10⁸ ufp de virus sont injectées aux souris de chacun des lots. Les souris destinées aux analyses biologiques sont prélevées dans les lots 3 jours après les injections.

On observe des résultats comparables à ceux reportés dans l'Exemple 2.

### EXEMPLE 4 : Traitement de souris DBA/2 porteuses de tumeurs par un virus de la vaccine comportant le gène codant pour le GM-CSF (VV-GM-CSF)

L'exemple 3 est répété en utilisant 10⁸ ufp de VV-GM-CSF. On enregistre l'évolution de la croissance de la tumeur au cours du temps comme indiqué dans l'exemple 1. Comme précédemment, on observe chez certains animaux du lot test, un ralentissement de la croissance des tumeurs par rapport aux lots témoins et au contrôle négatif. Trois souris sur 10 présentent une régression totale des tumeurs 15 jours après l'administration du vecteur viral.

## Revendications

1. Usage d'un vecteur viral dérivé d'un virus de la vaccine, dans le génome duquel est inséré un fragment d'ADN comportant un ou plusieurs gènes codant pour tout ou partie d'un agent modulateur de la réponse immunitaire et/ou inflammatoire sélectionné parmi les molécules co-stimulantes de surface cellulaire, pour la préparation d'un médicament pour le traitement d'un cancer chez les mammifères destiné à être administré par voie intraveineuse ou intramusculaire.

2. Usage d'un vecteur viral selon la revendication 1, selon lequel les molécules co-stimulantes de surface cellulaire sont sélectionnées parmi les antigènes de classes I et II du complexe majeur d'histocompatibilité (CMH), les ligands des marqueurs CD27, CD28, CD30 et CD40, l'antigène de fonction lymphocytaire de type 1 (LFA-1) et la molécule d'adhésion intercellulaire de type 1 (ICAM-1).

3. Usage d'un vecteur viral selon l'une des revendications 1 et 2, pour la préparation d'un médicament pour le traitement d'un cancer chez les humains.

4. Usage d'un vecteur viral selon l'une des revendications 1 à 3, pour la préparation d'un médicament pour le traitement d'une tumeur cancéreuse solide chez les mammifères.

5. Usage d'un vecteur viral selon l'une des revendications 1 à 4, selon lequel le vecteur viral comprend en outre un gène codant pour un antigène spécifique de tumeur.

6. Usage d'un vecteur viral dérivé d'un virus de la vaccine présentant un tropisme positif pour des cellules cancéreuses, dans le génome duquel est inséré un fragment d'ADN comportant un ou plusieurs gènes codant pour au moins un agent intervenant dans la destruction des cellules cancéreuses, placé sous le contrôle des éléments nécessaires à l'expression, pour la fabrication d'un médicament destiné à être administré par voie intraveineuse ou intramusculaire.

7. Usage d'un vecteur viral selon la revendication 6, dans le génome duquel est inséré un fragment d'ADN comportant un ou plusieurs gènes codant pour un agent modulateur de la réponse immunitaire et/ou inflammatoire, placé sous le contrôle des éléments nécessaires à l'expression, pour délivrer de manière spécifique ledit agent modulateur de la réponse immunitaire et/ou inflammatoire au niveau d'une tumeur cancéreuse.

8. Usage d'un vecteur viral selon la revendication 7, selon lequel ledit agent modulateur de la réponse immunitaire et/ou inflammatoire est sélectionné parmi les molécules co-stimulantes de surface cellulaire, les chémokines, les anticorps monoclonaux dirigés contre des marqueurs de surface lymphocytaire, les super-antigènes caractéristiques d'un organisme infectieux (bactérie, virus ou parasite), et les polypeptides à fonction d'adjuvant.

9. Usage d'un vecteur viral selon la revendication 8, selon lequel les molécules co-stimulantes de surface cellulaire sont sélectionnées parmi les antigènes de classes I et II du complexe majeur d'histocompatibilité (CMH), les ligands des marqueurs CD27, CD28, CD30 et CD40, l'antigène de fonction lymphocytaire de type 1 (LFA-1) et la molécule d'adhésion intercellulaire de type 1 (ICAM-1).

10. Usage d'un vecteur viral selon la revendication 8, selon lequel les chémokines sont sélectionnées parmi le PF-4 (Platelet Factor-4), le RANTES et l'ACT-2.

11. Usage d'un vecteur viral selon la revendication 1, selon lequel les anticorps monoclonaux sont sélectionnés parmi les anticorps monoclonaux anti-CD2, anti-CD3, anti-CD28 et anti-CD40.

12. Usage d'un vecteur viral selon la revendication 6, selon lequel ledit agent intervenant dans la destruction des cellules cancéreuses est une cytokine.

13. Usage d'un vecteur viral selon la revendication 12, selon lequel les cytokines sont sélectionnées parmi les interleukines, les interférons, les facteurs stimulant les colonies (CSF), les facteurs nécrosant les tumeurs (TNF), les facteurs inhibant la migration des macrophages (MIF) et le fragment C5a du complément.

14. Usage d'un vecteur viral selon la revendication 13, selon lequel les cytokines sont sélectionnées parmi les interleukines 2, 4, 5, 6 et 7, l'interféron gamma, le facteur stimulant les colonies de type granulocyte-macrophage (GM-CSF) et le facteur nécrosant les tumeurs de type β (TNFβ).

15. Usage d'un vecteur viral selon l'une des revendications 6 à 14, selon lequel le vecteur viral comprend en outre un gène codant pour un antigène spécifique de tumeur.

16. Usage d'un vecteur viral selon l'une des revendications 6 à 15, pour la préparation d'un médicament pour le traitement d'un cancer chez les humains.

17. Usage d'un vecteur viral selon l'une des revendications 6 à 16, pour la préparation d'un médicament pour le traitement d'une tumeur cancéreuse solide chez les mammifères.

## Patentansprüche

1. Verwendung eines von einem Vakziniavirus abgeleiteten viralen Vektors, in dessen Genom ein DNA-Fragment insertiert ist, das ein oder mehrere Gene, das bzw. die die Gesamtheit oder einen Teil eines Mittels zur Modulierung der Immun- und/oder Entzündungsantwort, ausgewählt unter den costimulierenden Zelloberflächenmolekülen, kodiert bzw. kodieren, umfasst, für die Herstellung eines Arzneimittels für die Behandlung einer Krebserkrankung bei Säugetieren, das dazu bestimmt ist, auf intravenösem oder intramuskulärem Wege verabreicht zu werden.

2. Verwendung eines viralen Vektors nach Anspruch 1, gemäß welcher die costimulierenden Zelloberflächenmoleküle unter den Antigenen der Klassen I und II des Haupthistokompatibilitätskomplexes (MHC), den Liganden der Marker CD27, CD28, CD30 und CD40, dem Lymphozytenfunktions-Antigen vom Typ 1 (LFA-1) und dem interzellulären Adhäsionsmolekül vom Typ 1 (ICAM-1) ausgewählt sind.

3. Verwendung eines viralen Vektors nach einem der Ansprüche 1 und 2 für die Herstellung eines Arzneimittels für die Behandlung einer Krebserkrankung beim Menschen.

4. Verwendung eines viralen Vektors nach einem der Ansprüche 1 bis 3 für die Herstellung eines Arzneimittels für die Behandlung eines festen krebsartigen Tumors bei Säugetieren.

5. Verwendung eines viralen Vektors nach einem der Ansprüche 1 bis 4, gemäß welcher der virale Vektor außerdem ein Gen, das ein spezifisches Tumorantigen kodiert, umfasst.

6. Verwendung eines von einem Vakziniavirus abgeleiteten viralen Vektors, der einen positiven Tropismus für Krebszellen aufweist, in dessen Genom ein DNA-Fragment, das ein oder mehrere Gene umfasst, das bzw. die wenigstens ein Mittel, das an der Zerstörung der Krebszellen beteiligt ist, kodiert bzw. kodieren, insertiert ist und unter die Kontrolle der für die Expression erforderlichen Elemente gestellt ist, für die Herstellung eines Arzneimittels, das dazu bestimmt ist, auf intravenösem oder intramuskulärem Wege verabreicht zu werden.

7. Verwendung eines viralen Vektors nach Anspruch 6, in dessen Genom ein DNA-Fragment, das ein oder mehrere Gene umfasst, das bzw. die ein Mittel zur Modulierung der Immunund/oder Entzündungsantwort kodiert bzw. kodieren, insertiert ist und unter die Kontrolle der für die Expression erforderlichen Elemente gestellt ist, um das Mittel zur Modulierung der Immun- und/oder Entzündungsantwort auf spezifische Weise auf der Ebene eines krebsartigen Tumors abzugeben.

8. Verwendung eines viralen Vektors nach Anspruch 7, gemäß welcher das Mittel zur Modulierung der Immun- und/oder Entzündungsantwort unter den costimulierenden Zelloberflächenmolekülen, den Chemokinen, den gegen Lymphozytenoberflächenmarker gerichteten monoklonalen Antikörpern, den für einen infektiösen Organismus (Bakterium, Virus oder Parasit) charakteristischen Superantigenen und den Polypeptiden mit Adjuvansfunktion ausgewählt ist.

9. Verwendung eines viralen Vektors nach Anspruch 8, gemäß welcher die costimulierenden Zelloberflächenmoleküle unter den Antigenen der Klassen I und II des Haupthistokompatibilitätskomplexes (MHC), den Liganden der Marker CD27, CD28, CD30 und CD40, dem Lymphozytenfunktions-Antigen vom Typ 1 (LFA-1) und dem interzellulären Adhäsionsmolekül vom Typ 1 (ICAM-1) ausgewählt sind.

10. Verwendung eines viralen Vektors nach Anspruch 8, gemäß welcher die Chemokine unter PF-4 (Platelet Factor-4; Plättchenfaktor-4), den RANTES und ACT-2 ausgewählt sind.

11. Verwendung eines viralen Vektors nach Anspruch 1, gemäß welcher die monoklonalen Antikörper unter den monoklonalen anti-CD2-, anti-CD3-, anti-CD28- und anti-CD40-Antikörpern ausgewählt sind.

12. Verwendung eines viralen Vektors nach Anspruch 6, gemäß welcher das Mittel, das an der Zerstörung der Krebszellen beteiligt ist, ein Zytokin ist.

13. Verwendung eines viralen Vektors nach Anspruch 12, gemäß welcher die Zytokine unter den Interleukinen, den Interferonen, den Kolonie-stimulierenden Faktoren (CSF), den Tumornekrosefaktoren (TNF), den Makrophagenmigrationsinhibitionsfaktoren (MIF) und dem Komplementfragment C5a ausgewählt sind.

14. Verwendung eines viralen Vektors nach Anspruch 13, gemäß welcher die Zytokine unter den Interleukinen 2, 4, 5, 6 und 7, Interferon-γ, dem Kolonie-stimulierenden Faktor vom Granulozyten-Makrophagen-Typ (GM-CSF) und dem Tumornekrosefaktor vom Typ β (TNFβ) ausgewählt sind.

15. Verwendung eines viralen Vektors nach einem der Ansprüche 6 bis 14, gemäß welcher der virale Vektor außerdem ein Gen, das ein spezifisches Tumorantigen kodiert, umfasst.

16. Verwendung eines viralen Vektors nach einem der Ansprüche 6 bis 15 für die Herstellung eines Arzneimittels für die Behandlung einer Krebserkrankung beim Menschen.

17. Verwendung eines viralen Vektors nach einem der Ansprüche 6 bis 16 für die Herstellung eines Arzneimittels für die Behandlung eines festen krebsartigen Tumors bei Säugetieren.

## Claims

1. Use of a viral vector derived from a vaccinia virus, into the genome of which is inserted a DNA fragment containing one or more genes coding for all or part of an agent that modulates the immune and/or inflammatory response, selected from cell surface costimulatory molecules, for the preparation of a medicinal product for the treatment of a cancer in mammals, intended to be administered intravenously or intramuscularly.

2. Use of a viral vector according to Claim 1, according to which the cell surface costimulatory molecules are selected from classes I and II antigens of the major histocompatibility complex (MHC), ligands for the markers CD27, CD28, CD30 and CD40, the lymphocyte function antigen type 1 (LFA-1) and the intercellular adhesion molecule type 1 (ICAM-1).

3. Use of a viral vector according to either of Claims 1 and 2, for the preparation of a medicinal product for the treatment of a cancer in humans.

4. Use of a viral vector according to one of Claims 1 to 3, for the preparation of a medicinal product for the treatment of a solid cancerous tumour in mammals.

5. Use of a viral vector according to one of Claims 1 to 4, according to which the viral vector comprises, in addition, a gene coding for a tumour-specific antigen.

6. Use of a viral vector derived from a vaccina virus displaying a positive tropism for cancer cells, into the genome of which is inserted a DNA fragment containing one or more genes coding for at least one agent participating in the destruction of cancer cells, placed under the control of the elements needed for expression, for the production of a medicinal product intended to be administered intravenously or intramuscularly.

7. Use of a viral vector according to Claim 6, into the genome of which is inserted a DNA fragment containing one or more genes coding for an agent that modulates the immune and/or inflammatory response, placed under the control of the elements needed for expression, to deliver the said agent that modulates the immune and/or inflammatory response specifically to a cancerous tumour.

8. Use of a viral vector according to Claim 7, according to which the said agent that modulates the immune and/or inflammatory response is selected from cell surface costimulatory molecules, chemokines, monoclonal antibodies directed against lymphocyte surface markers, superantigens characteristic of an infectious organism (bacterium, virus or parasite) and polypeptides having an adjuvant function.

9. Use of a viral vector according to Claim 8, according to which the cell surface costimulatory molecules are selected from classes I and II antigens of the major histocompatibility complex (MHC), ligands for the markers CD27, CD28, CD30 and CD40, the lymphocyte function antigen type 1 (LFA-1) and the intercellular adhesion molecule type 1 (ICAM-1).

10. Use of a viral vector according to Claim 8, according to which the chemokines are selected frog PF-4 (platelet factor-4), RANTES and ACT-2.

11. Use of a viral vector according to Claim 1, according to which the monoclonal antibodies are selected from anti-CD2, anti-CD3, anti-CD28 and anti-CD40 monoclonal antibodies.

12. Use of a viral vector according to Claim 6, according to which the said agent participating in the destruction of cancer cells is a cytokine.

13. Use of a viral vector according to Claim 12, according to which the cytokines are selected from interleukins, interferons, colony stimulating factors (CSF), tumour necrosis factors (TNF), macrophage migration inhibition factors (MIF) and complement fragment C5a.

14. Use of a viral vector according to Claim 13, according to which the cytokines are selected from interleukins-2, -4, -5, -6 and -7, gamma interferon, granulocyte-macrophage type colony stimulating factor (GM-CSF) and tumour necrosis factor type β (TNFβ).

15. Use of a viral vector according to one of Claims 6 to 14, according to which the viral vector comprises, in addition, a gene coding for a tumour-specific antigen.

16. Use of a viral vector according to one of Claims 6 to 15, for the preparation of a medicinal product for the treatment of a cancer in humans.

17. Use of a viral vector according to one of Claims 6 to 16, for the preparation of a medicinal product for the treatment of a solid cancerous tumour in mammals.
